# FASCICULE DE BREVET EUROPEEN

(11) **EP 0 611 175 B1**
(45) Date de publication et mention de la délivrance du brevet: **08.11.2000**
(21) Numéro de dépôt: 94400083.5
(22) Date de dépôt: 13.01.1994
(51) Int. Cl.: A61M 16/22

(54) **Cartouche d'adsorbant et unité d'adsorption pour système d'anesthésie**
Adsorbenspatrone und Adsorptionseinheit für eine Anästhesieeinrichtung
Adsorbent cartridge and adsorbtion unit for an anaesthesia system

(30) Priorité: 12.02.1993 FR 9301617
(43) Date de publication de la demande: 17.08.1994
(73) Titulaire: TAEMA, F-92182 Antony Cédex (FR)
(72) Inventeur: Jounenc, Michel, F-78180 Montigny Le Bretonneux (FR)
(74) Mandataire: Le Moenner, Gabriel

(56) Documents cités:
- DE-A- 3 219 444
- FR-A- 838 720
- US-A- 2 837 413
- US-A- 4 086 923
- US-A- 4 108 172
- US-A- 5 109 838

## Description

La présente invention concerne une unité d'adsorption destinée à être insérée dans un circuit patient d'un système d'anesthésie en circuit fermé, l'unité étant du type comprenant un conteneur contenant une dose d'adsorbant et comportant un fond et une extrémité ouverte, et des moyens de fixation du conteneur à une platine support définissant une portion du circuit patient, un conduit de gaz, raccordable au circuit patient, traversant l'adsorbant.

Une telle unité est décrite dans le document DE-A-32 19 444. Dans ce type d'unité, l'adsorbant est disposé en vrac dans le conteneur qui est plaqué sur la platine par un tube central solidarisant l'unité à la platine.

L'anesthésie en circuit fermé impose de pouvoir régénérer les gaz expirés par le patient avant réinhalation, l'unité d'adsorption étant destinée à piéger notamment le dioxyde de carbone expiré. Les unités actuelles ont une autonomie ne dépassant pas 8 heures d'anesthésie environ, ce qui impose de rebuter chaque jour l'adsorbant épuisé et de remplir l'unité avec une nouvelle dose d'adsorbant. La manipulation de cet adsorbant en vrac est difficile et délicate dans le contexte des blocs opératoires où l'espace est réduit et les conditions de propreté impératives.

Le document US-A-5 109 838 décrit par ailleurs une unité d'adsorption complexe avec une cartouche d'adsorbant positionnée dans un puits thermostaté d'un réceptacle surmonté d'un boîtier à circuits de valves connectable à un circuit patient.

La présente invention a pour objet de proposer une unité d'adsorption perfectionnée permettant, de façon simple et peu onéreuse, de faciliter grandement la mise en oeuvre des matériels d'anesthésie en augmentant considérablement l'ergonomie d'utilisation et en évitant les risques de dispersion de l'adsorbant.

Pour ce faire, selon l'invention, le conteneur est fixé par son extrémité ouverte à la platine support et la dose d'adsorbant est contenue dans une cartouche insérable dans le conteneur et comportant des faces axiales annulaires perméables aux gaz et un tube central raccordable à une portion du circuit patient.

D'autres caractéristiques et avantages de la présente invention ressortiront de la description suivante de modes de réalisation, donnée à titre illustratif mais nullement limitatif, faite en relation avec les dessins annexés, sur lesquels :
- la figure 1 est une vue schématique d'une unité d'adsorption selon l'invention, en configuration d'utilisation dans un circuit d'expiration d'un système d'anesthésie ; et
- la figure 2 est une vue à plus grande échelle d'une cartouche d'adsorbant selon l'invention.

Dans la description qui va suivre et sur les dessins, les éléments identiques ou analogues portent les mêmes chiffres de référence.

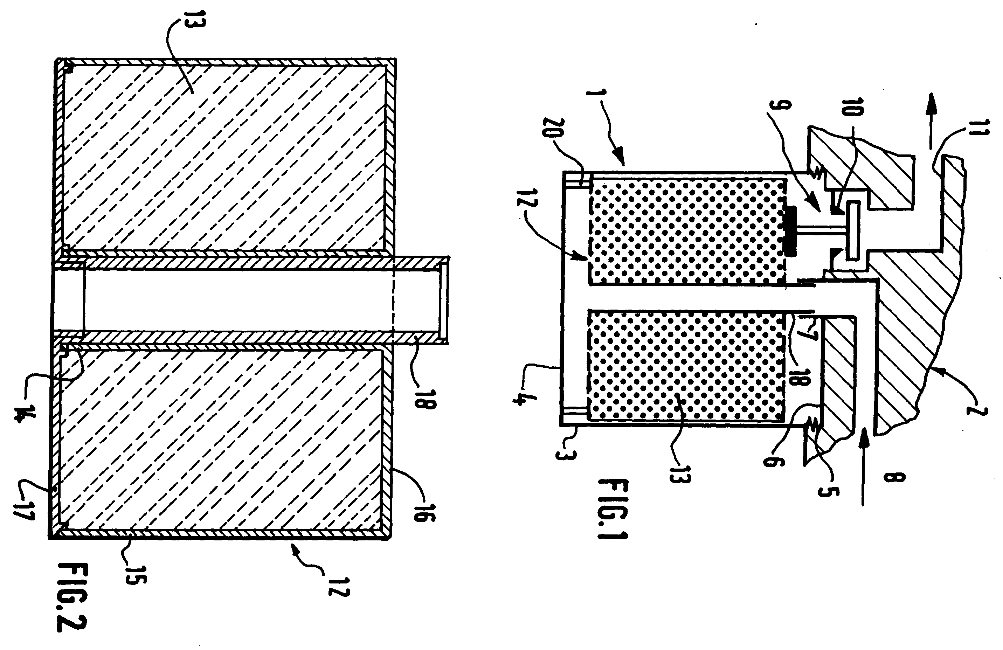

Sur la figure 1, on a représenté une unité d'adsorption, également appelée "canister", généralement désignée par la référence 1, qui est montée, suspendue, dans une platine-support 2 d'un bâti intermédiaire définissant une portion d'un circuit expiratoire d'un système d'anesthésie. Dans le mode de réalisation représenté, l'unité d'adsorption comporte un conteneur cylindrique 3 comportant un fond 4 et dont l'extrémité ouverte supérieure comporte un filetage 5 vissé dans un taraudage correspondant formé à la périphérie d'une chambre 6 ouverte vers le bas dans la platine 2. Dans la chambre 6 font saillie vers le bas un tube 7 constituant l'extrémité aval d'une partie amont 8 de circuit expiratoire, ainsi qu'un clapet 9 coopérant avec un siège 10 en amont d'une partie aval 11 du circuit expiratoire.

Selon un aspect de l'invention, dans le conteneur 3 est disposée, à coulissement juste, une cartouche cylindrique 12 contenant une quantité d'adsorbant 13, typiquement des granulés de chaux sodée occupant un volume d'environ 1,25 litre. La cartouche 12 comporte des parois annulaires concentriques intérieure 14 et extérieure 15, dont les extrémités axialement opposées sont fixées de façon étanche, par exemple par soudage, à des flasques d'extrémité annulaires perforés supérieur 16 et inférieur 17, les parois 14 à 17 définissant entre elles un volume dans lequel est confiné l'adsorbant 13. La cartouche 12 comporte en outre un tube ou fût central 18 faisant saillie, dans l'exemple représenté, par rapport au flasque annulaire supérieur 16 de façon à être reçu, lors de la mise en place de l'unité d'adsorption 1 dans la platine 2, à coulissement juste dans le tube 7. Lors de cette mise en place, le clapet 9, en appui contre le flasque annulaire supérieur 16, est soulevé vers le haut, à l'écart du siège 10, ouvrant ainsi le circuit de gaz expiratoire entre la portion de circuit 8, la cheminée centrale 18, le volume intérieur, défini dans l'unité 1, par exemple des entretoises 20, entre le fond 17 de la cartouche et le fond 4 du conteneur 3, la masse d'adsorbant 13, la chambre 6 et la portion de circuit aval 11.

On comprendra qu'avec l'agencement selon l'invention, pour changer l'adsorbant épuisé, il suffit de retirer l'unité d'adsorption 1 de la platine 2, ce qui provoque la fermeture du clapet 9, d'extraire la cartouche usée 13 pour la jeter, et de remettre en place une cartouche neuve, toutes ces opérations s'effectuant sans aucune manipulation de l'adsorbant. Le conteneur 1 est avantageusement en verre ou en matériau plastique rigide transparent, l'enveloppe et le fût central de la cartouche 12 étant avantageusement réalisés en matériau plastique thermosoudable, par exemple en polystyrène.

Quoique la présente invention ait été décrite en relation avec des modes de réalisation particuliers, elle ne s'en trouve pas limitée pour autant mais est au contraire susceptible de modifications et de variantes qui apparaîtront à l'homme de l'art.

## Revendications

1. Unité d'adsorption destinée à être insérée dans un circuit patient d'un système d'anesthésie en circuit fermé, l'unité (1) comprenant un conteneur (3) contenant une dose d'adsorbant (13) et comportant un fond (4) et une extrémité ouverte, et des moyens (5) de fixation du conteneur à une platine support (2) définissant une portion du circuit patient (8, 11), un conduit de gaz, raccordable au circuit patient, traversant l'adsorbant, caractérisée en ce que le conteneur (3) est fixé par son extrémité ouverte à la platine support (2) et an ce que la dose d'adsorbant est contenue dans une cartouche (12) insérable dans le conteneur (3) et comportant des faces axiales annulaires perméables aux gaz (16, 17) et un tube central (18) raccordable à une portion (8) du circuit patient.

2. Unité selon la revendication 1, caractérisée en ce que la cartouche (12) comporte une paroi périphérique étanche (15) raccordée à deux parois d'extrémité perforées (16, 17).

3. Unité selon la revendication 1 ou la revendication 2, caractérisée an ce que la cartouche (12) et le conteneur (3) sont cylindriques.

4. Unité selon la revendication 3, caractérisée en ce que l'extrémité ouverte du conteneur comporte un moyen de filetage (5) coopérant avec un moyen de taraudage dans la platine-support (2).

5. Unité selon la revendication 4, caractérisée an ce que la platine-support (2) comporte un clapet (9) disposé dans une portion aval (11) du circuit patient et coopérant, vers une position d'ouverture, avec la cartouche (12) lors du montage du conteneur (3) sur la platine (2).

6. Unité selon la revendication 4 ou la revendication 5, caractérisée an ce que le conteneur (3) est monté suspendu à la platine (2).

7. Unité selon l'une des revendications précédentes, caractérisée en ce que l'adsorbant (13) est constitué de granulés de chaux sodée.

8. Unité selon l'une des revendications précédentes, caractérisée en ce que le tube central (18) fait saillie par rapport à l'une (16) des faces axiales (16, 17) de la cartouche (12).

9. Unité selon la revendication 8, caractérisée en ce que le tube central (18) est reçu à coulissement dans une partie (7) de la portion amont du circuit patient (8).

10. Unité selon l'une des revendications précédentes, caractérisée an ce que le conteneur (3) est an verre ou matériau plastique rigide transparent.

## Claims

1. Adsorption unit intended to be inserted in a patient circuit of a closed-circuit anaesthesia system, the unit (1) comprising a container (3) containing a dose of adsorbent (13) and having a bottom (4) and an open end, and means (5) for securing the container to a support plate (2) that defines a portion of the patient circuit (8, 11), a gas pipe, which can be connected to the patient circuit, passing through the adsorbent, characterized in that the container (3) is secured to the support plate (2) by its open end and in that the dose of adsorbent is contained in a cartridge (12) that can be inserted into the container (3) and comprises gas-permeable annular axial faces (16, 17) and a central tube (18) connectable to a portion (8) of the patient circuit.

2. Unit according to Claim 1, characterized in that the cartridge (12) comprises a leak-tight peripheral wall (15) connected to two perforated end walls (16, 17).

3. Unit according to Claim 1 or Claim 2, characterized in that the cartridge (12) and the container (3) are cylindrical.

4. Unit according to Claim 3, characterized in that the open end of the container comprises a screw-threaded means (5) collaborating with a tapped means in the support plate (2).

5. Unit according to Claim 4, characterized in that the support plate (2) comprises a valve (9) placed in a downstream portion (11) of the patient circuit and collaborating, towards an open position, with the cartridge (12) when the container (3) is mounted on the plate (2).

6. Unit according to Claim 4 or Claim 5, characterized in that the container (3) is mounted such that it is suspended from the plate (2).

7. Unit according to one of the preceding claims, characterized in that the adsorbent (13) consists of soda lime granules.

8. Unit according to one of the preceding claims, characterized in that the central tube (18) projects with respect to one (16) of the axial faces (16, 17) of the cartridge (12).

9. Unit according to Claim 8, characterized in that the central tube (18) is housed with sliding in part (7) of the upstream portion of the patient circuit (8).

10. Unit according to one of the preceding claims, characterized in that the container (3) is made of transparent rigid plastic or glass.

## Patentansprüche

1. Adsorptionseinheit, die in einen Patientenkreis eines Anästhesiesystems mit geschlossenem Kreislauf eingesetzt werden soll, wobei die Einheit (1) einen Behälter (3), der eine Dosis eines Adsorbens (13) enthält und einen Boden (4) und ein offenes Ende besitzt, sowie Mittel (5) zur Befestigung des Behälters auf einer einen Teil des Patientenkreises (8, 11) darstellenden Halteplatte (2) umfaßt, wobei eine an den Patientenkreis anschließbare Gasleitung durch das Adsorbens führt, dadurch gekennzeichnet, daß der Behälter (3) mit seinem offenen Ende an der Halteplatte (2) befestigt ist und daß die Dosis Adsorbens in einer Patrone (12) enthalten ist, die in den Behälter (3) eingeführt werden kann und gasdurchlässige axiale ringförmige Flächen (16, 17) und ein Mittelrohr (18) besitzt, das an einen Teil (8) des Patientenkreises angeschlossen werden kann.

2. Einheit nach Anspruch 1, dadurch gekennzeichnet, daß die Patrone (12) eine dichte Umfangswand (15) besitzt, die mit zwei perforierten Endwänden (16, 17) verbunden ist.

3. Einheit nach Anspruch 1 oder Anspruch 2, dadurch gekennzeichnet, daß die Patrone (12) und der Behälter (3) zylindrisch sind.

4. Einheit nach Anspruch 3, dadurch gekennzeichnet, daß das offene Ende des Behälters ein Außengewinde (5) besitzt, das mit einem Innengewinde in der Halteplatte (2) zusammenwirkt.

5. Einheit nach Anspruch 4, dadurch gekennzeichnet, daß die Halteplatte (2) eine Klappe (9) umfaßt, die in einem hinteren Teil (11) des Patientenkreises angeordnet ist und in Richtung einer Öffnungsstellung bei der Montage des Behälters (3) auf der Platte (2) mit der Patrone (12) zusammenwirkt.

6. Einheit nach Anspruch 4 oder Anspruch 5, dadurch gekennzeichnet, daß der Behälter (3) hängend an der Platte (2) montiert ist.

7. Einheit nach einem der vorhergehenden Ansprüche, dadurch gekennzeichnet, daß das Adsorbens (13) aus einem Granulat kalkhaltiger Kreide besteht.

8. Einheit nach einem der vorhergehenden Ansprüche, dadurch gekennzeichnet, daß das Mittelrohr (18) in Bezug auf eine (16) der axialen Flächen (16, 17) der Patrone (12) vorsteht.

9. Einheit nach Anspruch 8, dadurch gekennzeichnet, daß das Mittelrohr (18) in einen Abschnitt (7) des vorderen Teils des Patientenkreises (8) eingeschoben wird.

10. Einheit nach einem der vorhergehenden Ansprüche, dadurch gekennzeichnet, daß der Behälter (3) aus Glas oder einem durchsichtigen, steifen Kunststoffmaterial besteht.
